# EUROPEAN PATENT APPLICATION

(11) **EP 4 582 107 A1**
(43) Date of publication of application: **09.07.2025**
(21) Application number: 23860510.9
(22) Date of filing: 31.08.2023
(51) Int. Cl.: A61K 48/00, A61P 13/12, A61K 38/17

(54) **AGENT FOR INHIBITING PROGRESSION OF UREMIA IN FELIDS WITH SEVERE RENAL FAILURE**

(30) Priority: 01.09.2022 JP 2022139482
(71) Applicant: Limited Liability Company Aimiya, Tokyo 162-0054 (JP)
(72) Inventor: MIYAZAKI, Toru, Tokyo 162-8666 (JP); ARAI, Satoko, Tokyo 162-8666 (JP)
(74) Representative: Weickmann & Weickmann PartmbB
(86) International application number: PCT/JP2023/031922
(87) International publication number: WO 2024/048748

(57) **Abstract**

The present invention provides an agent for suppressing deterioration of uremia in Felids suffering from severe renal failure, which contains an apoptosis inhibitor of macrophage (AIM), an AIM fragment having a biological activity of AIM, or a nucleic acid encoding the AIM or AIM fragment.

## Description

### [Technical Field]

The present invention relates to an agent for suppressing deterioration of uremia in Felids suffering from severe renal failure and the like. More particularly, the present invention relates to an agent for suppressing deterioration of uremia in Felids suffering from severe renal failure, which contains an apoptosis inhibitor of macrophage (AIM), and the like.

### [Background Art]

Apoptosis inhibitor of macrophage (AIM; also called CD5 antigen-like (CD5L)) is a blood protein produced by tissue macrophages, which the present inventors identified for the first time as a supporter of macrophage survival. AIM is now recognized as a molecule to induce repair processes in many diseases (Non Patent Literatures 1 to 3). AIM consists of three cysteine-rich domains (referred to as the SRCR domains), and a unique positively-charged amino-acid cluster is present at the carboxyl terminus within the third SRCR domain. This cluster forms charge-based interactions with dead cells that are strongly negatively charged due to the high-level exposure of phosphatidylserine on their surface (Non Patent Literatures 1, 2). This association highly enhances the engulfment of dead cells by phagocytes, as AIM is highly internalized by phagocytes via multiple scavenger receptors (Non Patent Literature 3).

In humans, AIM is generally present while bound to IgM pentamers. When acute kidney injury or the like occurs, it separates from the IgM pentamers to become free AIM, and binds to dead cells and debris to promote clearance thereof. This mechanism is conserved in many mammals. However, it is known that, in Felids, the binding between AIM and IgM pentamers is very strong and this mechanism does not function sufficiently (Non-Patent Literature 4). Therefore, dead cell debris in renal tissue is not cleared sufficiently in Felids. As a result, in Felids, dead cell debris accumulated in renal tissue with aging causes renal function to decline and eventually develops severe chronic renal failure, leading to uremia and death.

Considering such background, the present inventors have reported a method for treating or preventing renal diseases in humans and Felids by administering AIM (Patent Literature 1).

However, in the case of severe renal failure where most of the renal function has been lost, even if AIM is administered, recovery of renal function cannot be expected, and the progression of uremia cannot be suppressed. Accordingly, it was considered that there was no effective method for suppressing the onset and progression of uremia in Felids suffering from severe renal failure.

### [Citation List]

### [Patent Literature]

[Patent Literature 1]
WO 2015/119253

### [Non Patent Literature]

[Non Patent Literature 1]
   Arai, S. et al., Nat Med. 2016 Feb; 22(2):183-93.
[Non Patent Literature 2]
   Tomita, T. et al. Sci Rep. 2017 Jul 25; 7(1):6450.
[Non Patent Literature 3]
   Arai, S. and Miyazaki, T. Semin Immunopathol. 2018 Nov; 40 (6) : 567-575
[Non Patent Literature 4]
   Sugisawa R. et al., Sci Rep. 2016 Oct 12; 6:35251

### [Summary of Invention]

### [Technical Problem]

An object of the present invention is to provide a means capable of suppressing the deterioration of uremia in Felids suffering from severe renal failure.

### [Solution to Problem]

The present inventors have conducted intensive studies of the above-mentioned problems and found that when AIM is administered to cats with severe renal failure with no prospect of recovery of renal function, the concentrations of various uremic toxins in the blood are reduced, even though renal function does not improve, as a result of which the deterioration of uremia in cats suffering from severe renal failure can be suppressed. Based on such findings, they have conducted further studies and completed the present invention.

Accordingly, the present invention provides the following.

[1] An agent for suppressing deterioration of uremia in a Felid suffering from severe renal failure, comprising an apoptosis inhibitor of macrophage (AIM), an AIM fragment having a biological activity of AIM, or a nucleic acid encoding the AIM or AIM fragment, wherein the severe renal failure is defined by the blood indoxyl sulfate concentration which is 5 µg/mL or higher and the blood inorganic phosphorus concentration which is 7.5 mg/dL or lower.
[2] An agent for reducing the concentration of uremic toxins in the blood in a Felid suffering from severe renal failure, comprising an AIM, an AIM fragment having a biological activity of AIM, or a nucleic acid encoding the AIM or AIM fragment, wherein the severe renal failure is defined by the blood indoxyl sulfate concentration which is 5 µg/mL or higher and the blood inorganic phosphorus concentration which is 7.5 mg/dL or lower.
[3] An agent for reducing the concentration of uremic toxins in the blood in a mammal, comprising an AIM, an AIM fragment having a biological activity of AIM, or a nucleic acid encoding the AIM or AIM fragment.
[4] The agent of [3], wherein the mammal is a human, cow, monkey, horse, pig, sheep, goat, dog, cat, guinea pig, rat, mouse, rabbit, or hamster.
[5] The agent of [3], wherein the mammal is a human.
   [A1] A method for suppressing deterioration of uremia in a Felid suffering from severe renal failure, comprising administering an apoptosis inhibitor of macrophage (AIM), an AIM fragment having a biological activity of AIM, or a nucleic acid encoding the AIM or AIM fragment to the Felid suffering from severe renal failure, wherein the severe renal failure is defined by the blood indoxyl sulfate concentration which is 5 µg/mL or higher and the blood inorganic phosphorus concentration which is 7.5 mg/dL or lower.
   [A2] A method for reducing the concentration of uremic toxins in the blood in a Felid suffering from severe renal failure, comprising administering an AIM, an AIM fragment having a biological activity of AIM, or a nucleic acid encoding the AIM or AIM fragment to the Felid suffering from severe renal failure, wherein the severe renal failure is defined by the blood indoxyl sulfate concentration which is 5 µg/mL or higher and the blood inorganic phosphorus concentration which is 7.5 mg/dL or lower.
   [A3] A method for reducing the concentration of uremic toxins in the blood in a mammal, comprising administering an AIM, an AIM fragment having a biological activity of AIM, or a nucleic acid encoding the AIM or AIM fragment to the mammal.
   [A4] The method of [A3], wherein the mammal is a human, cow, monkey, horse, pig, sheep, goat, dog, cat, guinea pig, rat, mouse, rabbit, or hamster.
   [A5] The method of [A3], wherein the mammal is a human.
   [B1] An apoptosis inhibitor of macrophage (AIM), an AIM fragment having a biological activity of AIM, or a nucleic acid encoding the AIM or AIM fragment for use in suppressing the deterioration of uremia in a Felid suffering from severe renal failure, wherein the severe renal failure is defined by the blood indoxyl sulfate concentration which is 5 µg/mL or higher and the blood inorganic phosphorus concentration which is 7.5 mg/dL or lower.
   [B2] An AIM, an AIM fragment having a biological activity of AIM, or a nucleic acid encoding the AIM or AIM fragment for use in reducing the concentration of uremic toxins in the blood in a Felid suffering from severe renal failure, wherein the severe renal failure is defined by the blood indoxyl sulfate concentration which is 5 µg/mL or higher and the blood inorganic phosphorus concentration which is 7.5 mg/dL or lower.
   [B3] An AIM, an AIM fragment having a biological activity of AIM, or a nucleic acid encoding the AIM or AIM fragment for use in reducing the concentration of uremic toxins in a mammal.
   [B4] The AIM, AIM fragment having a biological activity of AIM, or nucleic acid encoding the AIM or AIM fragment of [B3], wherein the mammal is a human, cow, monkey, horse, pig, sheep, goat, dog, cat, guinea pig, rat, mouse, rabbit, or hamster.
   [B5] The AIM, AIM fragment having a biological activity of AIM, or nucleic acid encoding the AIM or AIM fragment of [B3], wherein the mammal is a human.
   [C1] Use of an AIM, an AIM fragment having a biological activity of AIM, or a nucleic acid encoding the AIM or AIM fragment in the production of a medicament for suppressing deterioration of uremia in a Felid suffering from severe renal failure, wherein the severe renal failure is defined by the blood indoxyl sulfate concentration which is 5 µg/mL or higher and the blood inorganic phosphorus concentration which is 7.5 mg/dL or lower.
   [C2] Use of an AIM, an AIM fragment having a biological activity of AIM, or a nucleic acid encoding the AIM or AIM fragment in the production of a medicament for reducing the concentration of uremic toxins in the blood in a Felid suffering from severe renal failure, wherein the severe renal failure is defined by the blood indoxyl sulfate concentration which is 5 µg/mL or higher and the blood inorganic phosphorus concentration which is 7.5 mg/dL or lower.
   [C3] Use of an AIM, an AIM fragment having a biological activity of AIM, or a nucleic acid encoding the AIM or AIM fragment in the production of a medicament for reducing the concentration of uremic toxins in the blood in a mammal.
   [C4] The use of [C3], wherein the mammal is a human, cow, monkey, horse, pig, sheep, goat, dog, cat, guinea pig, rat, mouse, rabbit, or hamster.
   [C5] The use of [C3], wherein the mammal is a human.

### [Advantageous Effects of Invention]

According to the present invention, deterioration of uremia can be suppressed in Felids suffering from severe renal failure with no prospect of recovery of renal function. According to the present invention, moreover, the life of Felids suffering from severe renal failure with no prospect of recovery of renal function can be prolonged. According to the present invention, the concentration of uremic toxins in the blood can be reduced in Felids suffering from severe renal failure with no prospect of recovery of renal function. According to the present invention, moreover, the concentration of uremic toxins in the blood can be reduced in mammals.

### [Brief Description of Drawings]

[Fig. 1]

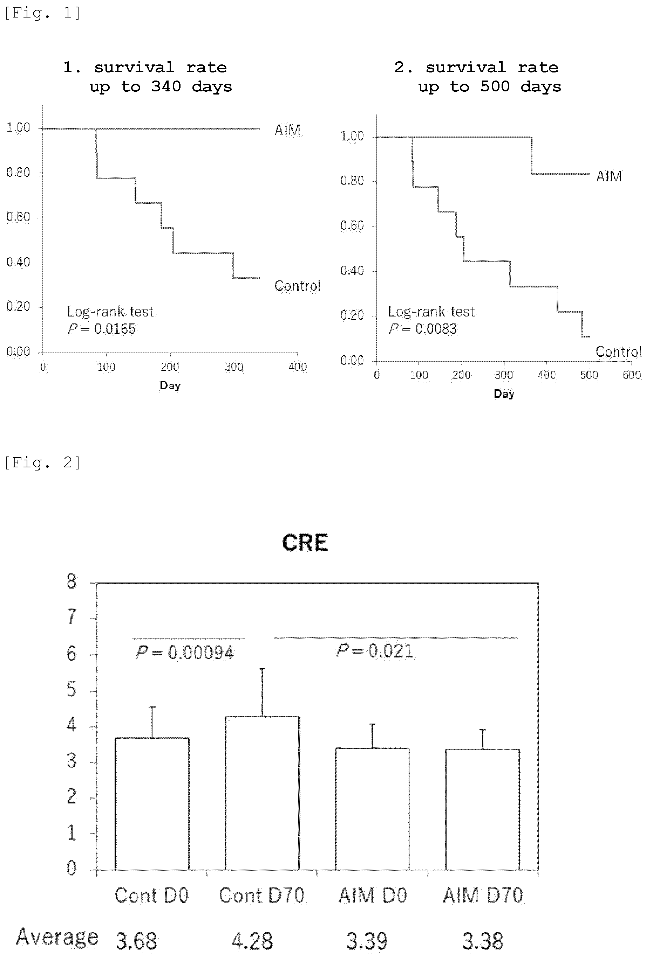

   Fig. 1 is a diagram showing the survival rate as a survival curve when mouse AIM was administered to cats suffering from severe renal failure (stage 3b). (Left) A survival curve up to 340 days was drawn because the shortest observation period was 340 days. (Right) Survival curve up to 500 days. For individuals with an observation period of less than 500 days, the survival curve was drawn assuming that they would survive for 500 days. The mean age at the start of administration was 14 years for the control group (n=9) and 16 years for the AIM-administered group (n=6).
[Fig. 2]
   Fig. 2 is a diagram showing the Cre (creatinine) concentrations in the blood in the AIM-administered group and the control group on Day0 and Day70. Error bars indicate S.D. Statistical analysis was performed using two-way repeated measures ANOVA and Bonferroni's post-hoc test.
[Fig. 3]

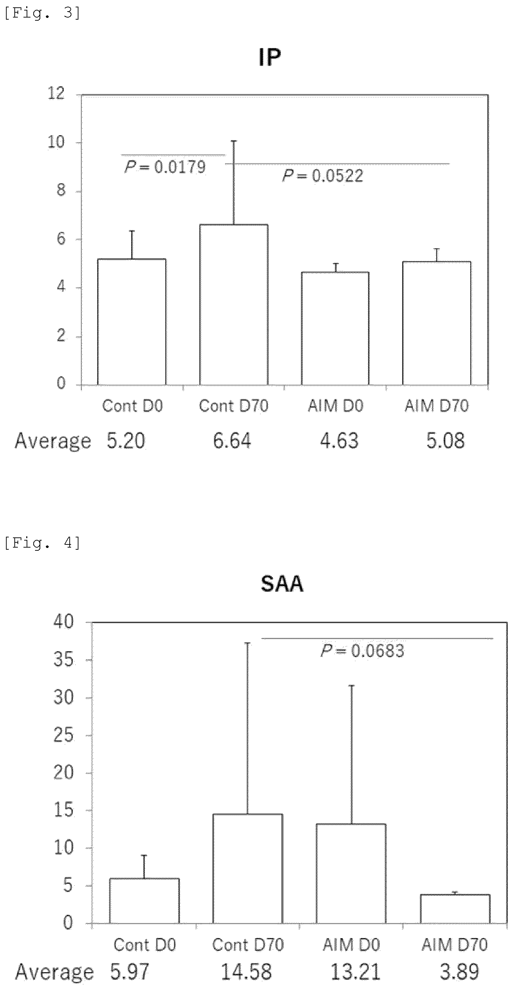

   Fig. 3 is a diagram showing the IP concentrations in the blood in the AIM-administered group and the control group on Day0 and Day70. Error bars indicate S.D. Statistical analysis was performed using two-way repeated measures ANOVA and Bonferroni's post-hoc test.
[Fig. 4]
   Fig. 4 is a diagram showing the SAA concentrations in the blood in the AIM-administered group and the control group on Day0 and Day70. Error bars indicate S.D. Statistical analysis was performed using two-way repeated measures ANOVA and Bonferroni's post-hoc test.
[Fig. 5]

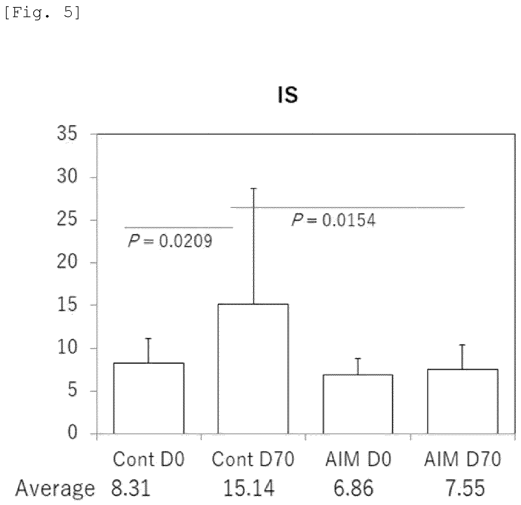

   Fig. 5 is a diagram showing the IS concentrations in the blood in the AIM-administered group and the control group on Day0 and Day70. Error bars indicate S.D. Statistical analysis was performed using two-way repeated measures ANOVA and Bonferroni's post-hoc test.
[Fig. 6]

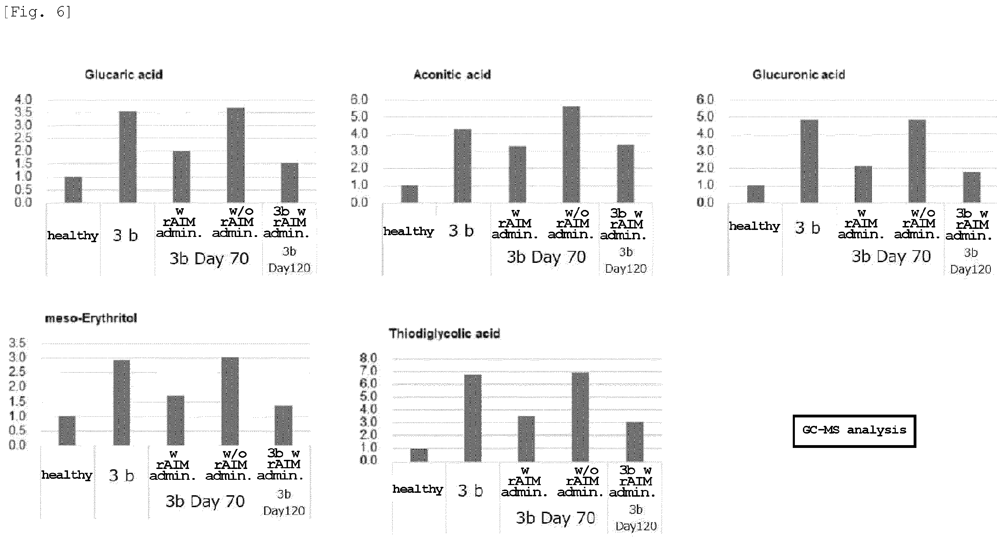

   Fig. 6 is a diagram showing the results of GC-Mass spectrometer analysis of serum from a healthy cat, serum from a stage 3b cat, serum from a stage 3b cat after AIM or PBS was administered intravenously a total of six times every two weeks (Day 70), and serum from a stage 3b cat 50 days after the completion of AIM administration (Day 120). This diagram shows glucaric acid, meso-Erythritol, thiodiglycolic acid, aconitic acid, and glucuronic acid as representative uremic toxins. The vertical axis shows fold change when the value in healthy cats is set to 1.
[Fig. 7]

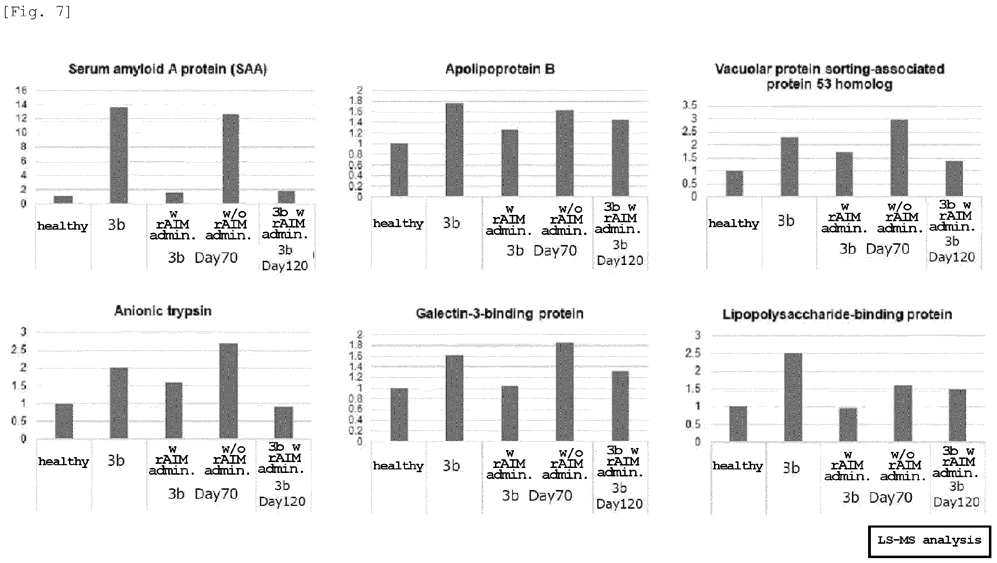

   Fig. 7 is a diagram showing the results of LC-Mass spectrometer analysis of serum from a healthy cat, serum from a stage 3b cat, serum from a stage 3b cat after AIM or PBS was administered intravenously a total of six times every two weeks (Day 70), and serum from a stage 3b cat 50 days after the completion of AIM administration (Day 120). This diagram shows only the results for SAA, which is a systemic inflammatory marker in cats, as well as Apolipoprotein B, Vacuolar protein sorting-associated protein 53 homolog, Anionic trypsin, Galectin-3-binding protein, and Lipopolysaccharide-binding protein. The vertical axis shows fold change when the value in healthy cats is set to 1.
[Fig. 8]

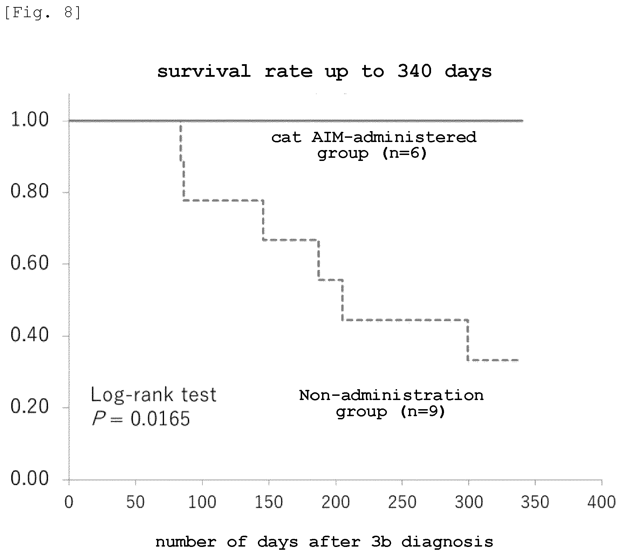

   Fig. 8 is a diagram showing the survival rate as a survival curve when cat AIM was administered to cats suffering from severe renal failure (stage 3b).
[Fig. 9]

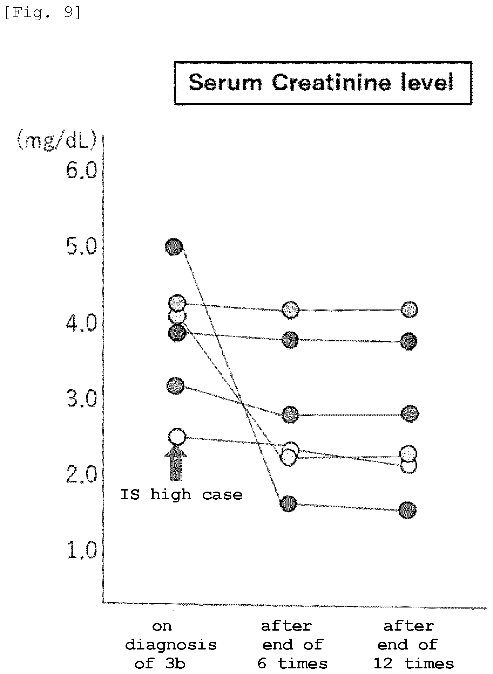

   Fig. 9 is a diagram showing the time-course changes in blood Cre levels when cat AIM was administered to cats suffering from severe renal failure (stage 3b).

### [Description of Embodiments]

The present invention is described in detail in the following.

### [Definitions]

### [severe renal failure]

In the present specification, "severe renal failure" in a Felid is defined by "the blood indoxyl sulfate concentration of 5 µg/mL or higher and the blood inorganic phosphorus concentration of 7.5 mg/dL or lower in a Felid". In one embodiment, severe renal failure in a Felid may be defined by "the blood indoxyl sulfate concentration of 5 µg/mL or higher and the blood inorganic phosphorus concentration of 6.8 mg/dL or lower in a Felid". Felids suffering from renal failure defined as such are generally in a state where majority (about 80-90%) of renal function is lost.

The "the blood indoxyl sulfate concentration of 5 µg/mL or higher and the blood inorganic phosphorus concentration of 7.5 mg/dL or lower in a Felid" and the "the blood indoxyl sulfate concentration of 5 µg/mL or higher and the blood inorganic phosphorus concentration of 6.8 mg/dL or lower in a Felid" are both conditions corresponding to late stage 3 in the staging of chronic kidney disease in Felids (especially Felis catus) provided by the International Renal Interest Society (IRIS). In the IRIS staging, a condition in which about 95% of renal function has been lost is classified as stage 4.

### [Felids]

In the present specification, a "Felid" means an animal classified in the Felidae. Examples of the Felid include, but are not limited to, Panthera leo, Panthera pardus, Panthera tigris, Panthera uncia, Panthera onca, Neofelis nebulosa, Neofelis diardi, Puma concolor, Acinonyx jubatus, Herpailurus yagouaroundi, Caracal aurata, Caracal caracal, Leptailurus serval, Catopuma badia, Catopuma temminckii, Pardofelis marmorata, Leopardus colocola, Leopardus geoffroyi, Leopardus guigna, Leopardus guttulus, Leopardus jacobita, Leopardus pardalis, Leopardus tigrinus, Leopardus wiedii, Lynxcanadensis, Lynx lynx, Lynx pardinus, Lynx rufus, Prionailurus bengalensis, Prionailurus javanensis, Prionailurus planiceps, Prionailurus rubiginosus, Prionailurus viverrinus, Otocolobus manul, Felis bieti, Felis catus, Felis chaus, Felis lybica, Felis margarita, Felis nigripes, Felis silvestris, and subspecies thereof. In a preferred embodiment of the present invention, the animal classified as Felidae is Felis catus.

### [Uremic toxin]

In the present specification, "uremic toxin" refers to various molecules whose concentrations in the blood increase as chronic kidney disease/renal failure progresses. Examples of the "uremic toxin" in the present specification include, but are not limited to, indoxyl sulfate, inorganic phosphorus, glucaric acid, meso-Erythritol, thiodiglycolic acid, aconitic acid, glucuronic acid, 2-hydroxyisovaleric acid, galacturonic acid, homogentisic acid, inositol, urea, phosphonic acid, arabinose, mannose, arabitol, serum amyloid A protein (SAA), apolipoprotein B, KIAA0100, Vacuolar protein sorting-associated protein 53 homolog, anionic trypsin, IgH variable region, galectin-3-binding protein, Ig lambdavariable region, coagulation factor IX, phosphoglycerate mutase, and the like.

### 1. Agent for suppressing deterioration of uremia in a Felid suffering from severe renal failure

The present invention provides an agent for suppressing deterioration of uremia in a Felid suffering from severe renal failure, which contains an apoptosis inhibitor of macrophage (AIM), an AIM fragment having a biological activity of AIM, or a nucleic acid encoding the AIM or AIM fragment (hereinafter sometimes referred to as "the agent for suppressing deterioration of uremia of the present invention").

AIM to be contained in the agent for suppressing deterioration of uremia of the present invention is a protein containing an amino acid sequence that is the same or substantially the same as the amino acid sequence shown in SEQ ID NO: 1 (amino acid sequence of human-derived AIM protein). AIM may be, for example, a protein isolated and purified from macrophage, which is immunocyte of warm-blooded animals (e.g., cat, mouse, human, rat, rabbit, sheep, pig, cow, horse, dog, monkey, chimpanzee, bird, and the like). It may also be a protein chemically synthesized or biochemically synthesized in a cell-free translation system. Alternatively, the protein may be a recombinant protein produced from a transformant incorporating a nucleic acid containing a base sequence that encodes the above-described amino acid sequence. The biological species from which the AIM to be contained in the agent for suppressing deterioration of uremia of the present invention is derived may be the same as or different from the biological species of a Felid to be its application target. For example, when the application target of the agent for suppressing deterioration of uremia of the present invention is a cat, the AIM contained in the agent for suppressing deterioration of uremia of the present invention may be cat AIM or mouse AIM.

Substantially the same amino acid sequence as the amino acid sequence shown in SEQ ID NO:1 refers to an amino acid sequence having an identity or similarity of about 60% or more, preferably about 70% or more, further preferably about 80% or more, particularly preferably about 90% or more, most preferably about 95% or more, to the amino acid sequence shown in SEQ ID NO:1, and the like. As used herein, the "identity" means the proportion (%) of the same amino acids and similar amino acid residues to the overlapping total amino acid residues in the optimal alignment (preferably, the algorithm can consider, for the optimal alignment, introduction of a gap into one or both of the sequences), when two amino acid sequences are aligned using mathematical algorithm known in the technical field. The "similarity" means the ratio (%) of the number of positions where the same or similar amino acid residues are present in two amino acid sequences when they are aligned to the total number of amino acid residues. The "similar amino acid" means amino acids similar in physicochemical properties and, for example, amino acids classified into the same group such as aromatic amino acid (Phe, Trp, Tyr), aliphatic amino acid (Ala, Leu, Ile, Val), polar amino acid (Gln, Asn), basic amino acid (Lys, Arg, His), acidic amino acid (Glu, Asp), amino acid having hydroxyl group (Ser, Thr), amino acid with small side chain (Gly, Ala, Ser, Thr, Met) and the like can be mentioned. Substitution with such similar amino acids is predicted to cause no change in the phenotype of the protein (i.e., conservative amino acid substitution). Specific examples of the conservative amino acid substitution are well known in the art and described in various documents (see, for example, Bowie et al., Science, 247:1306-1310(1990)).

The identity or similarity of the amino acid sequence in the present specification can be calculated using the identity or similarity calculation algorithm NCBI BLAST (National Center for Biotechnology Information Basic Local Alignment Search Tool) under the following conditions (expectancy=10; gap; matrix =BLOSUM62; filtering=OFF). Examples of other algorithm to determine the identity or similarity of amino acid sequence include the algorithm described in Karlin et al., Proc. Natl. Acad. Sci. USA, 90:5873-5877(1993) [the algorithm is incorporated in NBLAST and XBLAST program (version 2.0) (Altschul et al., Nucleic Acids Res., 25:3389-3402(1997))], the algorithm described in Needleman et al., J. Mol. Biol., 48:444-453(1970) [the algorithm is incorporated in GAP program in GCG software package], the algorithm described in Myers and Miller, CABIOS, 4:11-17(1988) [the algorithm is incorporated in ALIGN program (version 2.0) which is a part of the CGC sequence alignment software package], the algorithm described in Pearson et al., Proc. Natl. Acad. Sci. USA, 85: 2444-2448(1988) [the algorithm is incorporated in FASTA program in the GCG software package] and the like, and they can also be preferably used in the same manner. More preferably, substantially the same amino acid sequence as the amino acid sequence shown in SEQ ID NO:1 is an amino acid sequence having an identity of about 60% or more, preferably about 70% or more, further preferably about 80% or more, particularly preferably about 90% or more, and most preferably about 95% or more, to the amino acid sequence shown in SEQ ID NO:1.

As a protein containing substantially the same amino acid sequence as the amino acid sequence shown in SEQ ID NO:1, for example, a protein containing substantially the same amino acid sequence as the aforementioned amino acid sequence shown in SEQ ID NO:1, and having substantially the same quality of activity as the biological activity of wild-type AIM can be mentioned. Examples of the biological activity of the wild-type AIM include endocytosis activity on macrophage (including microglia), activity to suppress apoptosis of macrophage, activity to maintain or promote arteriosclerosis, adipocyte differentiation suppressive activity, activity to melt lipid droplet of adipocyte, adipocyte reducing activity, CD36 binding activity, endocytosis activity to adipocyte, FAS binding activity, FAS function suppressive activity, antiobesity activity, activity to prevent or treat hepatic diseases (fatty liver, NASH, cirrhosis, liver cancer), activity to prevent or treat renal diseases (acute renal failure, chronic nephritis, chronic renal failure, nephrotic syndrome, diabetic nephropathy, nephrosclerosis, IgA nephropathy, hypertensive nephropathy, nephropathy with collagen disease or IgM nephropathy) and the like. In the present invention, particularly, an endocytosis activity on macrophage can be a preferable index. The activity can be confirmed using in vitro macrophage (or microglia) phagocytosis tests, though not limited thereto. In the present specification, the "substantially the same quality" means that the activity thereof is qualitatively (e.g., physiologically or pharmacologically) the same. Therefore, it is preferable that the aforementioned activities be equivalent to each other, but the quantitative factors of these activities, such as the extent of activity (e.g., about 0.1 to about 10 times, preferably about 0.5 to about 2 times) and the molecular weight of the protein, may be different. The aforementioned activities can be measured by a method known per se.

Examples of the AIM to be used in the present invention also include proteins containing (1) an amino acid sequence having 1 or 2 or more (preferably about 1 to 100, preferably about 1 to 50, further preferably about 1 to 10, particularly preferably 1 to several (2, 3, 4 or 5)) amino acids deleted from the amino acid sequence shown in SEQ ID NO:1, (2) an amino acid sequence having 1 or 2 or more (preferably about 1 to 100, preferably about 1 to 50, further preferably about 1 to 10, particularly preferably 1 to several (2, 3, 4 or 5)) amino acids added to the amino acid sequence shown in SEQ ID NO:1, (3) an amino acid sequence having 1 or 2 or more (preferably about 1 to 50, preferably about 1 to 10, further preferably 1 to several (2, 3, 4 or 5)) amino acids inserted in the amino acid sequence shown in SEQ ID NO:1, (4) an amino acid sequence having 1 or 2 or more (preferably about 1 to 50, preferably about 1 to 10, further preferably 1 to several (2, 3, 4 or 5)) amino acids substituted by other amino acids in the amino acid sequence shown in SEQ ID NO:1, or (5) an amino acid sequence containing a combination thereof. When an amino acid sequence has been inserted, deleted or substituted as described above, the position of the insertion, deletion or substitution is not particularly limited as long as a desired biological activity (e.g., endocytosis activity on macrophage (including microglia)) of the protein is maintained.

AIM in the present invention is preferably a cat AIM protein having the amino acid sequence shown in SEQ ID NO:1, or a homologue thereof in other mammals [for example, mouse homologue registered in the GenBank as Accession No.: AAD01445 (SEQ ID NO: 3) and the like], more preferably, a cat AIM protein consisting of the amino acid sequence shown in SEQ ID NO:1. Cats have two types of AIM (three-domain type (SRCR1-SRCR2-SRCR3, SEQ ID NO: 1) and four-domain type (SRCR1-SRCR1-SRCR2-SRCR3)), and either may be used.

In the present specification, the protein and peptide are described according to conventional peptide lettering, and the left end is N-terminus (amino terminus), and the right end is C-terminus (carboxyl terminus). In AIM to be used in the present invention including a protein containing the amino acid sequence shown in SEQ ID NO:1, the C-terminus may be any of a carboxyl group (-COOH), carboxylate (-COO⁻), amide (-CONH₂) and ester (-COOR).

Here, as R in the ester, a C₁₋₆ alkyl group, for example, methyl, ethyl, n-propyl, isopropyl and n-butyl, a C₃₋₈ cycloalkyl group, for example, cyclopentyl and cyclohexyl, a C₆₋₁₂ aryl group, for example, phenyl and α-naphthyl, a phenyl-C₁₋₂ alkyl group, for example, benzyl and phenethyl, a C₇₋₁₄ aralkyl group, for example, an α-naphthyl-C₁₋₂ alkyl group, for example, α-naphthylmethyl, a pivaloyloxymethyl group; and the like can be used.

When the AIM to be used in the present invention has a carboxyl group (or carboxylate) at a position other than the C-terminus, a protein wherein the carboxyl group is amidated or esterified is also included in the protein of the present invention. In this case, as the ester, the above-described ester at the C terminus, and the like, for example, is used.

Furthermore, the AIM to be used in the present invention also includes a protein wherein the amino group of the N-terminal amino acid residue is protected by a protecting group (e.g., formyl group, acetyl group, and the like); a protein wherein the glutamine residue that may be produced upon cleavage at the N terminus in vivo has been converted to pyroglutamic acid, a protein wherein a substituent (e.g., -OH, -SH, amino group, imidazole group, indol group, guanidino group and the like) on a side chain of an amino acid in the molecule is protected by an appropriate protecting group (e.g., C₁₋₆ acyl groups such as C₁₋₆ alkanoyl groups such as formyl group and acetyl group, and the like), a conjugated peptide such as what is called a glycopeptide having a sugar chain bound thereto, and the like.

In the present specification, the "AIM" is a concept including not only wild-type AIM but also mutants of these and the like having an activity substantially the same or improved than the biological activity of the wild-type AIM. Here, the "activity of substantially the same quality" is as defined above. In addition, the "activity of substantially the same quality" can be measured in the same manner as in the case of AIM.

As a component of the agent for suppressing deterioration of uremia of the present invention, not only AIM but also an AIM fragment having the biological activity of AIM can also be used. Whether or not the AIM fragment has the biological activity of wild-type AIM may be determined by the aforementioned method.

As one embodiment of an AIM fragment, since intact AIM protein contains 3 SRCR (Scavenger-Receptor Cysteine-Rich) domains containing a large amount of cysteine, each SRCR domain can be recited as an example of an AIM fragment having substantially the same quality of activity as the biological activity possessed by the wild-type AIM. To be specific, for example, of the amino acid sequence shown in SEQ ID NO:5 (human AIM), partial amino acid sequences respectively containing SRCR1 domain (amino acid Nos. 24 - 125 of the amino acid sequence shown in SEQ ID NO:5), SRCR2 domain (amino acid Nos. 139 - 239 of the amino acid sequence shown in SEQ ID NO:5), and SRCR3 domain (amino acid Nos. 244 - 346 of the amino acid sequence shown in SEQ ID NO:5), partial amino acid sequence containing any combination of SRCR domains and the like can be used as the AIM fragment. The size of the AIM fragment having substantially the same quality of activity as the biological activity possessed by the wild-type AIM is not particularly limited as long as it contains the above-mentioned functional domain. The partial peptide typically contains a partial amino acid sequence of 20 or more amino acids, preferably a partial amino acid sequence of 50 or more amino acids, more preferably a partial amino acid sequence of 100 or more amino acids, further preferably a partial amino acid sequence of 200 or more amino acids. The partial amino acid sequences may be a single consecutive partial amino acid sequence, or discontinuous plural partial amino acid sequences linked to each other. The SRCR domain of human AIM is used here for explanation, but the SRCR domain of AIM of other animal species can also be used.

In addition, the C-terminus of the AIM fragment to be used in the present invention may be any of a carboxyl group (-COOH) , carboxylate (-COO⁻), amide (-CONH₂) and ester (-COOR). Here, examples of the R in ester include those similar to the examples recited above for AIM. When the partial peptide of the present invention has a carboxyl group (or carboxylate) at a position other than the C-terminus, the carboxyl group may be amidated or esterified, which is also encompassed in the partial peptide of the present invention. As the ester in this case, for example, those similar to the ester at the C-terminus and the like are used.

Furthermore, the AIM fragment to be used in the present invention includes, in the same manner as in the above-mentioned AIM, the amino group of the N-terminal amino acid residue may be protected with a protecting group, the glutamine residue at the N-terminus may be converted to pyroglutamic acid, a substituent on the side chain of the amino acid in a molecule may be protected with an appropriate protecting group, or the partial peptide may be a conjugated peptide such as what is called glycopeptide having a sugar chain bound thereto, and the like.

The AIM (including AIM fragment) to be used in the present invention may be in the form of a salt. For example, salts with physiologically acceptable acid (e.g., inorganic acid, organic acid), base (e.g., alkali metal salt) and the like are used, and a physiologically acceptable acid addition salt is particularly preferable. Examples of such salt to be used include salts with inorganic acids **(e.g.,** hydrochloric acid, phosphoric acid, hydrobromic acid, sulfuric acid), salts with organic acids **(e.g.,** acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

AIM (including AIM fragments) can also be produced according to a known method of peptide synthesis. The method of peptide synthesis may be any of, for example, a solid phase synthesis method and a liquid phase synthesis method. A desired protein can be produced by condensing a partial peptide or amino acid capable of constituting AIM with the remaining portion, and removing any protecting group the resultant product may have.

Here, the condensation and the protecting group removal are conducted in accordance with methods known per se, for example, the methods indicated in (1) and (2) below:
(1) M. Bodanszky and M.A. Ondetti: Peptide Synthesis, Interscience Publishers, New York (1966)
(2) Schroeder and Luebke: The Peptide, Academic Press, New York (1965).

AIM thus obtained can be purified or isolated by a known method of purification. Here, as examples of the method of purification, solvent extraction, distillation, column chromatography, liquid chromatography, recrystallization, combinations thereof and the like can be mentioned.

When AIM obtained by the above-mentioned method is in a free form, the free form can be converted into a suitable salt form by a known method or a method based thereon, and on the other hand, when the AIM is obtained in the form of a salt, it can be converted into a free form or in the form of a different salt by a known method or a method based thereon.

Furthermore, AIM can also be produced by culturing a transformant containing a nucleic acid encoding the same, and separating and purifying AIM from the obtained culture. The nucleic acid encoding AIM or AIM fragment may be DNA or RNA, or DNA/RNA chimera, preferably DNA. In addition, the nucleic acid may be double-stranded or single-stranded. In the case of a double-stranded nucleic acid, it may be a double-stranded DNA, a double-stranded RNA, or a DNA:RNA hybrid. In the case of a single strand, it may be a sense strand (that is, coding strand), or an antisense strand (that is, non-coding strand).

Examples of the DNA encoding AIM (including AIM fragments) include genomic DNA, cDNA derived from macrophage of warm-blooded animal (e.g., human, cow, monkey, horse, pig, sheep, goat, dog, cat, guinea pig, rat, mouse, rabbit, hamster, bird and the like), synthetic DNA and the like. Genomic DNA encoding AIM or an AIM fragment can be directly amplified by Polymerase Chain Reaction (hereinafter to be abbreviated as "PCR method") by using, as a template, a genomic DNA fraction prepared from any cell of the aforementioned animals [for example, hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immunocyte (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or corresponding progenitor cell, stem cell or cancer cell thereof, and the like], or any tissue where such cells are present [for example, brain or any portion of the brain (e.g., olfactory bulb, amygdaloid nucleus, basal ganglia, hippocampus, thalamus, hypothalamus, subthalamic nucleus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue (e.g., brown adipose tissue, white adipose tissue), skeletal muscle and the like], and cDNA encoding AIM or an AIM fragment can also be directly amplified by PCR method and Reverse Transcriptase-PCR (hereinafter to be abbreviated as "RT-PCR method") by using, as a template, a total RNA or mRNA fraction prepared from macrophage, respectively. Alternatively, the genomic DNA and cDNA encoding AIM or a peptide fragment thereof can also be cloned by colony or plaque hybridization method or PCR method and the like from a genomic DNA library and cDNA library prepared by inserting the above-mentioned genomic DNA and total RNA or a fragment of mRNA into a suitable vector. The vector used for the library may be any of a bacteriophage, a plasmid, a cosmid, a phagemid and the like.

Examples of the nucleic acid encoding AIM include a nucleic acid containing the same or substantially the same base sequence as the base sequence shown in SEQ ID NO: 2 and the like. As the nucleic acid containing the same or substantially the same base sequence as the base sequence shown in SEQ ID NO: 2, for example, a nucleic acid containing a base sequence having an identity or similarity of about 60% or more, preferably about 70% or more, more preferably about 80% or more, and particularly preferably about 90% or more, with the base sequence shown in SEQ ID NO: 2, and encoding a protein having an activity of substantially the same quality as the aforementioned AIM and the like can be used. In one embodiment, the nucleic acid containing the same or substantially the same base sequence as the base sequence shown in SEQ ID NO: 2 is a nucleic acid containing a base sequence having an identity of about 60% or more, preferably about 70% or more, more preferably about 80% or more, and particularly preferably about 90% or more, with the base sequence shown in SEQ ID NO: 2, and encoding a protein having the same or substantially the same quality of activity as the aforementioned AIM. The nucleic acid encoding AIM also includes a nucleic acid sequence subjected to codon optimization for the purpose of increasing expression efficiency in an organism to be the application target.

The identity or similarity of the base sequence in the present specification can be calculated under the following conditions (an expectation value = 10; gaps are allowed; filtering = ON, match score=1; mismatch score=-3) using an identity or similarity scoring algorithm NCBI BLAST (National Center for Biotechnology Information Basic Local Alignment Search Tool). As other algorithm for determining the identity or similarity of the base sequence, for example, the above-mentioned homology calculation algorithm for amino acid sequences can be similarly preferably recited as the example.

The nucleic acid encoding AIM is preferably a nucleic acid containing a base sequence encoding cat AIM protein shown by the base sequence shown in SEQ ID NO: 2, or a homologue thereof in other mammals [for example, mouse homologue registered in GenBank as accession No: AF011428 (SEQ ID NO: 4) and the like].

As the component of the agent for suppressing deterioration of uremia of the present invention, a nucleic acid encoding AIM, or an AIM fragment having a biological activity of AIM can also be used.

The nucleic acid encoding AIM or AIM fragment to be used in the present invention may be any as long as it contains a base sequence encoding a peptide containing the same or substantially the same amino acid sequence as a part of the amino acid sequence shown in SEQ ID NO:1. Specifically, as a nucleic acid encoding the AIM fragment, (1) a nucleic acid containing a partial base sequence of the base sequence shown in SEQ ID NO: 2, or (2) a nucleic acid containing a base sequence having an identity or similarity of about 60% or more, preferably about 70% or more, more preferably about 80% or more, and particularly preferably about 90% or more, with a nucleic acid containing a partial base sequence of the base sequence shown in SEQ ID NO: 2, and encoding a protein having an activity of substantially the same quality as the aforementioned AIM and the like are used.

The nucleic acid encoding AIM or AIM fragment can be cloned by amplifying same using a synthesized DNA primer having a part of a base sequence encoding the AIM or AIM fragment by PCR method, or by conducting hybridization of a DNA incorporated into a suitable expression vector with a labeled DNA fragment or synthetic DNA encoding a part or whole region of AIM. Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under highly stringent conditions.

As examples of the highly stringent conditions, conditions of a hybridization reaction in 6×SSC (sodium chloride/sodium citrate) at 45°C followed by washing in 0.2×SSC/0.1% SDS at 65°C once or more and the like can be mentioned. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature and the like as appropriate. When a commercially available library is used, hybridization can be conducted according to the method described in the instruction manual attached to the library.

The nucleic acid encoding AIM or AIM fragment may also be functionally linked to an expression vector or the like having a suitable promoter. In addition to the promoter, known sequences such as Poly A addition signal, Kozak consensus sequence, tag sequence, linker sequence, NLS and the like may also be mounted as appropriate into a viral vector and the like together with the nucleic acid encoding AIM or AIM fragment according to the purpose.

In one preferable embodiment, a nucleic acid encoding AIM or AIM fragment is mounted on a viral vector. Preferable examples of the viral vector include, but are not limited to, adeno-associated virus, adenovirus, lentivirus, and Sendai virus. In consideration of the use in gene therapy, adeno-associated virus is preferable because it can express transgene for a long period of time, is highly safe because it is derived from a non-pathogenic virus and the like. In addition, the serotype of the adeno-associated virus is not particularly limited as long as the desired effect of the present invention can be obtained, and any of serotypes 1, 2, 3, 4, 5, 6, 7, 8, 9, and 10 may also be used. The viral vector to be used in the present invention also includes variants thereof. As a variant of a viral vector, one with a modified capsid and the like are known. Particularly, examples of the AAV variant include, but are not limited to, those disclosed in WO 2012/057363 and the like.

A viral vector containing a nucleic acid encoding AIM or AIM fragment can be prepared by a known method. In brief, a plasmid vector for virus expression is prepared by inserting a nucleic acid encoding AIM or AIM fragment and, if necessary, a nucleic acid having a desired function, this is transfected into an appropriate host cell to transiently produce a viral vector containing the polynucleotide of the present invention, and the viral vector is recovered.

For example, when AAV vector is prepared, a vector plasmid is first constructed in which the ITRs at both ends of a wild-type AAV genome sequence are left and, in the place of the DNA encoding the Rep proteins and capsid proteins, a nucleic acid encoding AIM or AIM fragment is inserted. On the other hand, the DNAs encoding Rep proteins and capsid proteins, which is necessary for forming virus particles, are inserted into another plasmid. Furthermore, a plasmid containing genes (E1A, E1B, E2A, VA, and E4orf6) responsible for the adenovirus helper action necessary for the proliferation of AAV is prepared as an adenovirus helper plasmid. These three plasmids are co-transfected into a host cell, whereby recombinant AAV (i.e., AAV vector) is produced within the cell. As the host cell, it is preferable to use a cell (e.g., 293 cell, etc.) that can supply a part of the gene product(s) (proteins) of the gene(s) responsible for the aforementioned helper action, and when such a cell is used, it is not necessary to mount the gene encoding the protein(s) that can be supplied from the host cell on the aforementioned adenovirus helper plasmid. Since the produced AAV vector exists in the nucleus, the host cell is frozen and thawed and recovered, and separated and purified by density ultracentrifugation method using cesium chloride, column method, and the like, whereby the desired AAV vector is prepared.

The route of administration of the agent for suppressing deterioration of uremia of the present invention is not particularly limited. Examples of the preferred administration route include, but are not limited to, intravenous administration, intraarterial administration, subcutaneous administration, intraperitoneal administration, and the like.

When the agent for suppressing deterioration of uremia of the present invention is formulated for parenteral administration, for example, it can be formulated as injection, suppository or the like. The injection may include the dosage forms of intravenous injection, subcutaneous injection, intradermal injection, intramuscular injection, drip injection, and the like. Such injection can be prepared according to a known method. Injection can be prepared, for example, by dissolving, suspending or emulsifying components such as AIM, nucleic acid encoding AIM and/or virus mounting a nucleic acid encoding AIM and the like in a sterile aqueous solution or an oily solution generally used for injection. As the aqueous solution for injection, for example, saline, isotonic solution containing glucose and other auxiliary agents, and the like are used, which may be used in combination with a suitable solubilizing agent, for example, alcohol (e.g. ethanol), polyalcohol (e.g., propylene glycol, polyethylene glycol), nonionic surfactant [e.g., polysorbate80, HCO-50 (polyoxyethylene (50 mol) adduct of hydrogenated castor oil)] and the like. As the oily solution, sesame oil, soybean oil and the like are used and, as a solubilizing agent, benzyl benzoate, benzyl alcohol and the like may be used in combination. A prepared injection is preferably filled in a suitable ampoule.

The dose of the agent for suppressing deterioration of uremia of the present invention to a subject is not particularly limited as long as the desired effect can be obtained, and may be appropriately optimized according to the kind and form of the active ingredient, the age and body weight of the subject, the administration schedule, the administration method, and the like.

The timing of administering the agent for suppressing deterioration of uremia of the present invention to a subject is not particularly limited as long as the desired effect can be obtained.

The agent for suppressing deterioration of uremia of the present invention may be used in combination with other therapeutic agents and dietary therapies to treat severe renal failure.

The agent for suppressing deterioration of uremia of the present invention can suppress the deterioration of uremia in Felids suffering from severe renal failure. In the present specification, "uremia" refers to a condition in which metabolic waste products (described in detail below as "uremic toxins") that should be excreted in urine accumulate in the blood due to decline in renal function. Examples of the symptoms of uremia include, but are not limited to:
Central nervous system: impaired consciousness, insomnia, memory loss, headache, confusion, convulsions, and the like Peripheral nervous system: peripheral neuropathy, discomfort in legs, and the like
Gastrointestinal tract: loss of appetite, vomiting, abdominal distension, and the like
Lungs: difficulty breathing, pulmonary edema, hypoxemia, and the like
Heart or blood vessels: palpitations, high blood pressure, and the like
Metabolism: elevated potassium, acidosis, and the like
Eyes: impaired vision, and the like
Blood: anemia, and the like
Skin: subcutaneous bleeding, edema, pigmentation, itching, and the like

In the present specification, "suppressing the deterioration of uremia" means that the symptoms of uremia observed at a certain time point in a Felid suffering from severe renal failure do not progress to a worse state after the lapse of a certain time period. "Suppressing the deterioration of uremia" can also be rephrased as "suppressing the progression of uremia".

Furthermore, in the present specification, "suppressing the deterioration of uremia" includes not only not deteriorating the symptoms of uremia, but also slowing the rate of deterioration of uremia. Therefore, the agent for suppressing deterioration of uremia of the present invention can be used not only for the purpose of not deteriorating uremia in a subject, but also for the purpose of slowing the deterioration of uremia in a subject. In addition, in one embodiment, "suppressing the deterioration of uremia" also includes suppressing the onset of uremia. Therefore, the agent for suppressing deterioration of uremia of the present invention can be used not only for the purpose of not developing uremia in a subject, but also for the purpose of slowing the timing of onset of uremia in a subject.

Incidentally, it has been reported that when uremia in Felids progresses with the progression of severe renal failure and reaches a state classified as IRIS stage 4, they die within several months (average 103 days) on average. However, in cats in IRIS late stage 3 to which the agent for suppressing deterioration of uremia of the present invention has been applied, the transition to IRIS stage 4 is suppressed, and as a result, the number of days of survival is drastically extended. In consideration of such situation, the present invention can be said to enable the life extension of Felids suffering from severe renal failure. Therefore, in one embodiment, the agent for suppressing deterioration of uremia of the present invention can be rephrased as a life-prolonging agent for Felids suffering from severe renal failure.

Furthermore, as shown in the following Examples, administration of AIM to a cat suffering from severe renal failure can reduce the concentration of uremic toxins in the blood of the cat, without recovery of renal function. Therefore, the present invention also provides an agent for reducing the concentration of uremic toxins in the blood of Felids suffering from severe renal failure, which contains an apoptosis inhibitor of macrophage (AIM), an AIM fragment having a biological activity of AIM, or a nucleic acid encoding the AIM or AIM fragment (hereinafter sometimes to be referred to as "the uremic toxin-reducing agent of the present invention").

The AIM and the like to be used in the uremic toxin-reducing agent of the present invention and the Felids and the like to be the application target are the same as those described for the agent for suppressing deterioration of uremia of the present invention.

In the present specification, "uremic toxin" refers to substances that are generally filtered by the glomeruli of the kidney and excreted in urine. In the present specification, "uremic toxin" is a concept that encompasses not only substances that have a direct adverse effect on the living body, but also substances that do not have a direct adverse effect on the living body but show increased blood concentration as a result of decreased renal function (e.g., precursors of substances having toxicity, etc.). Examples of the uremic toxin whose blood concentration can be reduced by the uremic toxin-reducing agent of the present invention include, but are not limited to, indoxyl sulfate, inorganic phosphorus, glucaric acid, meso-Erythritol, thiodiglycolic acid, aconitic acid, glucuronic acid, 2-hydroxyisovaleric acid, galacturonic acid, homogentisic acid, inositol, urea, phosphonic acid, arabinose, mannose, arabitol, SAA (serum amyloid A protein), apolipoprotein B, KIAA0100, Vacuolar protein sorting-associated protein 53 homolog, anionic trypsin, IgH variable region, galectin-3-binding protein, Ig lambda variable region, coagulation factor IX, phosphoglycerate mutase, and the like. By administration of the uremic toxin-reducing agent of the present invention, it is possible to reduce the blood concentrations of at least one, preferably at least two, at least three, at least four, at least five, at least six, at least seven, at least eight, at least nine, at least ten, more preferably at least fifteen, at least twenty, at least twenty-five, further preferably all of these uremic toxins.

Based on the fact that the administration of AIM to a cat suffering from severe renal failure can reduce the concentration of uremic toxins in the blood without improving renal function, it is clear that the reduction in the concentration of uremic toxins in the blood by the administration of AIM is due to a mechanism that does not involve renal function. Therefore, it is considered that AIM directly promotes the excretion of uremic toxins through some mechanism. Given these facts, it is considered that the blood uremic toxin-reducing action of AIM does not depend on the state of renal failure or the animal species. Therefore, in one embodiment, the present invention provides an agent for reducing the concentration of uremic toxins in the blood in mammals, which contains an apoptosis inhibitor of macrophage (AIM), an AIM fragment having a biological activity of AIM, or a nucleic acid encoding the AIM or AIM fragment (hereinafter sometimes to be referred to as "the uremic toxin-reducing agent for mammals of the present invention").

The mammal to which the uremic toxin-reducing agent for mammals of the present invention can be applied is not particularly limited as long as it is a mammal in which reduction of uremic toxins by AIM can be realized. Examples of mammals include, but are not limited to, human, cow, monkey, horse, pig, sheep, goat, dog, cat, guinea pig, rat, mouse, rabbit, hamster, and the like.

The AIM and the like to be used in the uremic toxin-reducing agent for mammals of the present invention are the same as those described for the agent for suppressing deterioration of uremia of the present invention.

In one preferred embodiment, the uremic toxin-reducing agent for mammals of the present invention can be applied to mammals suffering from end-stage renal failure to the extent that dialysis is required. By administering the uremic toxin-reducing agent for mammals of the present invention, the concentration of uremic toxins in the blood of the mammal can be reduced, making it possible to reduce the frequency of dialysis. In this embodiment, the mammal is preferably a human or a dog, particularly preferably a human. For humans, it is preferable to use human AIM (SEQ ID NO: 5) or a nucleic acid encoding human AIM (SEQ ID NO: 6). As described above, these may be mutants as long as they retain the biological activity of AIM.

For example, one embodiment of a mutant of the human AIM is, but not limited to, the following.

The mutant human AIM of the present invention preferably contains the amino acid sequence of any one of the following (1b) to (5b).
(1b) an amino acid sequence wherein cysteine at amino acid number 191 of the amino acid sequence shown in SEQ ID NO: 5 is substituted with serine,
(2b) an amino acid sequence wherein cysteine at amino acid number 300 of the amino acid sequence shown in SEQ ID NO: 5 is substituted with serine,
(3b) an amino acid sequence wherein cysteine at amino acid number 191 of the amino acid sequence shown in SEQ ID NO: 5 is substituted with serine, and an amino acid sequence wherein cysteine at amino acid number 300 of the amino acid sequence shown in SEQ ID NO: 5 is substituted with serine,
(4b) an amino acid sequence substantially the same as the amino acid sequence of any one of (1b) to (3b), and cysteines present in the amino acid sequence of any one of (1b) to (3b) and the substituted serine remain,
(5b) an amino acid sequence further containing deletion, addition, insertion or substitution of one to several amino acids or a combination thereof at a position other than cysteines present in the amino acid sequence of any one of (1b) to (3b) and the substituted serine.

As AIM mutants having functions equivalent to or improved over wild-type recombinant AIM, those disclosed in Japanese Patent No. 7231230 and the like can be used.

In another embodiment, the uremic toxin-reducing agent for mammals of the present invention can also be administered to mammals suffering from mild or moderate renal failure. By administering the uremic toxin-reducing agent for mammals of the present invention to said mammals, the reduced renal function can be assisted.

### 2. Method for suppressing deterioration of uremia in Felids suffering from severe renal failure

The present invention provides a method for suppressing deterioration of uremia in Felids suffering from severe renal failure, including administering an apoptosis inhibitor of macrophage (AIM), an AIM fragment having a biological activity of AIM, or a nucleic acid encoding the AIM or AIM fragment to the Felids suffering from severe renal failure (hereinafter sometimes to be referred to as "the uremia deterioration-suppressing method of the present invention").

The uremia deterioration-suppressing method of the present invention is achieved by administering the agent for suppressing deterioration of uremia of the present invention to Felids suffering from severe renal failure. The AIM and the like, application target, administration timing and the like in the uremia deterioration-suppressing method of the present invention are the same as those described for the agent for suppressing deterioration of uremia of the present invention.

In one embodiment, the uremia deterioration-suppressing method of the present invention can be rephrased as a life-prolonging method for Felids suffering from severe renal failure.

In addition, the present invention provides a method for reducing the concentration of uremic toxins in the blood in Felids suffering from severe renal failure, including administering the uremic toxin-reducing agent of the present invention to the Felids (hereinafter sometimes referred to as "the uremic toxin-reducing method of the present invention").

In another embodiment, the present invention provides a method for reducing the concentration of uremic toxins in the blood in mammals, including administering the uremic toxin-reducing agent of the present invention to the mammals.

The present invention is explained more specifically in the following Examples; however, the present invention is not limited in any way by these Examples.

### [Example]

### [Example 1] Administration of AIM to cats suffering from severe renal failure

AIM was administered to cats suffering from severe renal failure, and influence thereof was confirmed. The cats used in this test (n=15) met the following conditions:
(1) blood indoxyl sulfate (IS) concentration of 5 µg/mL or higher, and
(2) blood inorganic phosphorus (IP) concentration of 7.5 mg/dL or lower.

Indoxyl sulfate is known as one of uremic toxins and is a known marker for renal failure in cats. The condition of renal failure defined by (1) and (2) means a renal failure state corresponding to the late stage 3 in IRIS staging (in other words, a state in which most (about 90%) of renal function has been lost and moderate uremia has developed). In the present specification, the renal failure state that satisfies the conditions (1) and (2) is sometimes referred to as "stage 3b".

Fifteen cats classified as stage 3b were divided into a control group (n=9) and an AIM-administered group (n=6). The AIM-administered group received 2 mg/day of recombinant mouse AIM via slow intravenous infusion over one to several hours. Using this as one course, and AIM was administered once every two weeks, a total of six times, until the cat died. On the other hand, the control group did not receive AIM. The survival rates of the AIM-administered group and control group are shown in Fig. 1.

As shown in Fig. 1, the survival rate of the AIM-administered group was remarkably improved compared to the control group.

Furthermore, the measurement results of Cre (renal function marker), IP (uremic toxin), SAA (inflammation marker), and IS (uremic toxin) in each group on observation day 0 (D0) and observation day 70 (D70) are shown in Figs. 2 to 5.

As shown in Fig. 2, even with AIM administration, no change was found in the Cre value on D0 and D70. This means that administration of AIM suppresses further deterioration of renal function, but does not improve renal function.

On the other hand, administration of AIM suppressed an increase in the blood concentrations of uremic toxins IP and IS, even though renal function was not recovered (Fig. 3 and Fig. 5).

In addition, the concentration of SAA, which is an inflammatory marker, was significantly reduced by the administration of AIM (Fig. 4).

From the above, it was shown that the administration of AIM to cats suffering from severe renal failure at stage 3b can remarkably reduce the concentration of uremic toxins in the blood of the cats suffering from severe renal failure, even though the renal function cannot be improved, and, as a result, deterioration of uremia can be suppressed.

### [Example 2] Reduction of uremic toxins by AIM administration

Mixtures of serum samples from six healthy cats, six stage 3b cats, and six stage 3b cats after intravenous administration of recombinant mouse AIM (2 mg) or PBS every two weeks, six times in total (Day 70), or three cats at 50 days after the end of recombinant mouse AIM administration (Day 120), were analyzed by GC-mass spectrometer (MS) and LC-MS.

The GC-MS and LC-MS analyses were performed using the following methods and conditions.

### [GC-MS analysis method]

Among the 401 kinds of components (568 kinds of compounds if isomers of methoximes or TMS derivatives are included) listed in the database (SHIMADZU Smart Metabolites Database), compounds whose peaks were detected in the sample solution were picked up. The peak areas of these compounds were divided by the peak area of IS (2-isopropylmalate-3TMS) to calculate the IS correction value. For comparison between groups, the IS corrected value of each compound in healthy cats was set to 1, and the relative values of each group were graphed.

### [LC-MS TMT comparison analysis conditions]

Comparative analysis was performed on peptides with unique sequences in each protein (unique peptide) and razor peptides (*) as targets. The protein abundance ratio between each sample was normalized by dividing by the median abundance ratio of all proteins and the results are shown. To compare between groups, the value of healthy cats was set to 1, and the relative values of each group were graphed. (*Peptides shared between proteins are assigned as those derived from the protein with the highest number of identified peptides, and these are called razor peptides.)

Representative results obtained by GC-MS and LC-MS are shown in Fig. 6 and Fig. 7.

Many uremic toxins increased (accumulated) remarkably in the blood of stage 3b cats, and the increase was suppressed and reduced after administration of recombinant mouse AIM (Day 70). These uremic toxins were confirmed to be substances that also increase in the blood of human patients with end-stage renal failure who undergo dialysis (data not shown). On the other hand, accumulation was maintained or even increased in the PBS-administered group (Day 70). In cats administered with recombinant mouse AIM, low values of these factors were maintained even 50 days after the end of administration (Day 120) (since many of the 3b cats in the PBS-administered group died within 120 days after the start of the test, analysis on Day 120 was not performed). Similar results were obtained with SAA, which is a marker of systemic inflammation.

### [Example 3] Administration of AIM to cats suffering from severe renal failure - 2

Recombinant cat AIM was administered to cats suffering from severe renal failure, and influence thereof was confirmed.

Six cats classified as stage 3b received recombinant cat AIM at 2 mg/head administered intravenously (AIM-administered group). Using this as one course, AIM was administered once every two weeks and 12 times. On the other hand, the control group did not receive AIM (control group was the same as that obtained in Example 1). The survival rates (Kaplan-Meier curve) of the AIM-administered group and control group are shown in Fig. 8. Moreover, the time-course changes in serum creatinine value in the six cats that received AIM administration are shown in Fig. 9.

As shown in Fig. 8, the survival rate of the AIM-administered group was remarkably improved compared to the control group. As shown in Fig. 9, even with AIM administration, no change was found in the blood Cre value in most cats. This means that administration of AIM suppresses further deterioration of renal function, but does not improve renal function.

These results demonstrate that administration of AIM to cats in stage 3b can reduce the concentration of uremic toxins in the blood and suppress inflammation extremely efficiently.

Not wishing to be bound by theory, since the administration of AIM to stage 3b cats did not improve renal function, it is possible that the reduction of uremic toxins in the blood by AIM is due to a direct action of AIM on uremic toxins, thereby promoting the excretion of the uremic toxins from the blood. It is thus highly likely that the mechanism by which AIM reduces the concentration of uremic toxins in the blood is not limited by the stage of renal failure or cats. Therefore, the reduction of uremic toxins in the blood by the administration of AIM is applicable to all mammals with AIM, and the stage of renal failure is not limited.

### [Industrial Applicability]

According to the present invention, deterioration of uremia can be suppressed in Felids suffering from severe renal failure with no prospect of recovery of renal function. According to the present invention, moreover, the life of Felids suffering from severe renal failure with no prospect of recovery of renal function can be prolonged. According to the present invention, the concentration of uremic toxins in the blood can be reduced in Felids suffering from severe renal failure with no prospect of recovery of renal function. Therefore, the present invention is extremely useful in the field of veterinary medicine. In addition, according to the present invention, moreover, the concentration of uremic toxins in the blood can be reduced in mammals. Therefore, for example, the present invention can reduce the frequency of dialysis in human end-stage renal failure patients requiring dialysis. Hence, the present invention is also extremely useful in the field of human medicine.

This application is based on a patent application No. 2022-139482 filed in Japan (filing date: September 1, 2022), the contents of which are incorporated in full herein.

## Claims

1. An agent for suppressing deterioration of uremia in a Felid suffering from severe renal failure, comprising an apoptosis inhibitor of macrophage (AIM), an AIM fragment having a biological activity of AIM, or a nucleic acid encoding the AIM or AIM fragment, wherein the severe renal failure is defined by the blood indoxyl sulfate concentration which is 5 µg/mL or higher and the blood inorganic phosphorus concentration which is 7.5 mg/dL or lower.

2. An agent for reducing the concentration of uremic toxins in the blood in a Felid suffering from severe renal failure, comprising an apoptosis inhibitor of macrophage (AIM), an AIM fragment having a biological activity of AIM, or a nucleic acid encoding the AIM or AIM fragment, wherein the severe renal failure is defined by the blood indoxyl sulfate concentration which is 5 µg/mL or higher and the blood inorganic phosphorus concentration which is 7.5 mg/dL or lower.

3. An agent for reducing the concentration of uremic toxins in the blood in a mammal, comprising an apoptosis inhibitor of macrophage (AIM), an AIM fragment having a biological activity of AIM, or a nucleic acid encoding the AIM or AIM fragment.

4. The agent according to claim 3, wherein the mammal is a human, cow, monkey, horse, pig, sheep, goat, dog, cat, guinea pig, rat, mouse, rabbit, or hamster.

5. The agent according to claim 3, wherein the mammal is a human.
